(19) Europäisches Patentamt
European Patent Office
Office européen des brevets

(11) Veröffentlichungsnummer: **0 050 739**
**B1**

# (12) EUROPÄISCHE PATENTSCHRIFT

(45) Veröffentlichungstag der Patentschrift: **02.11.83**

(51) Int. Cl.³: **C 07 C 118/00**

(21) Anmeldenummer: **81107479.8**

(22) Anmeldetag: **21.09.81**

## (54) Verfahren zur Herstellung aliphatischer Isocyanate.

(30) Priorität: **29.10.80 DE 3040692**

(43) Veröffentlichungstag der Anmeldung: **05.05.82 Patentblatt 82/18**

(45) Bekanntmachung des Hinweises auf die Patenterteilung: **02.11.83 Patentblatt 83/44**

(84) Benannte Vertragsstaaten: **BE CH DE FR GB LI**

(56) Entgegenhaltungen:
**DE - A - 1 593 642**
**US - A - 3 275 669**
**US - A - 3 372 180**

(73) Patentinhaber: **BASF Aktiengesellschaft, Carl-Bosch-Strasse 38, D-6700 Ludwigshafen (DE)**

(72) Erfinder: **Schwendemann, Volker, Dr., Hauptstrasse 64 A, D-6901 Wiesenbach (DE)**
Erfinder: **Mangold, Dietrich, Dr., Hermann-Walker-Strasse 49, D-6903 Neckargemuend (DE)**

**EP 0 050 739 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung aliphatischer Isocyanate

Die Erfindung betrifft ein Verfahren zur Herstellung von organischen Isocyanaten durch thermische Zersetzung von N,N'-disubstituierten Allophansäureestern in organischen Carbonaten bei 150 bis 400°C.

Es ist bekannt, Isocyanate durch thermische Dehydrohalogenierung von Carbaminsäurehalogeniden darzustellen. Die aus Amin und Phosgen zugänglichen Carbaminsäurehalogenide werden bei erhöhter Temperatur in die entsprechenden Isocyanate und Chlorwasserstoff gespalten. Dieses Syntheseprinzip ist jedoch praktisch auf solche Isocyanate beschränkt, deren Siedepunkte über den Zersetzungstemperaturen der Carbaminsäurehalogenide liegen [R. J. Slocombe, JACS 72, 1888–1891 (1950)].

Für die Reihe der niederen Isocyanate trifft dies jedoch nicht zu. Hier tritt beim Erreichen der Zersetzungstemperatur des Carbaminsäurehalogenids das überhitzte Isocyanat in den Dampfraum und bildet an der kältesten Stelle der Apparatur mit Chlorwasserstoff in exothermer Reaktion das Ausgangsmaterial zurück [L. Gattermann, Ann. 244, 35 (1888)].

Ebenso ist bekannt, Isocyanate durch thermische Zersetzung von Carbaminsäureestern oder Harnstoffen darzustellen [Houben-Weyl, Methoden der Organischen Chemie, Band 8, Seite 126 (1952)].

Zur Pyrolyse von Harnstoffen geeignet sind trisubstituierte Harnstoffe mit höchstens zwei Arylgruppen (DE-PS 748 714), wobei Bedingung ist, dass die beiden Amidgruppierungen der verwendeten Harnstoffe zwei Aminen von erheblich verschiedenem Siedepunkt entsprechen.

Zur thermischen Zersetzung von Carbaminsäureestern verwendet man vorteilhaft Carbaminsäurearylester, welche im Vergleich zu Carbaminsäurealkylester bereits unter milderen Bedingungen in Isocyanat und Hydroxykomponente gespalten werden. Auch reagieren Isocyanate mit aliphatischen Hydroxykomponenten wesentlich rascher zu Carbamaten ab als mit den entsprechenden aromatischen Hydroxyverbindungen.

Nachteilig bei allen diesen Verfahren ist der hohe Verbrauch des äusserst toxischen Phosgens, das erhebliche Sicherheitsvorkehrung notwendig macht. Bei all diesen Verfahren wird pro Mol des zu bildenden Isocyanats selbst bei stöchiometrischem Umsatz mindestens ein Mol Phosgen verbraucht.

Diesen Nachteil versuchen mehrere Verfahren dadurch zu umgehen, dass als Ausgangsverbindung zur Darstellung von Alkylisocyanaten N,N'-disubstituierte Harnstoffe verwendet werden, die sehr einfach aus Kohlendioxid und dem entsprechenden Amin zugänglich sind.

So können nach US-PS 3 275 669 die aus Phosgen und N,N'-Dialkylharnstoffen zugänglichen Allophansäurechloride vorzugsweise unter Verwendung von Säureakzeptoren in zwei Mol Isocyanat übergeführt werden. Für die Reihe der niederen Alkylisocyanate treten jedoch wieder die gleichen Probleme auf, die bereits bei der Isocyanatherstellung aus Carbaminsäurehalogeniden geschildert wurden. Zusätzlich bewirkt die Verwendung von Basen eine verstärkte Polymerisation der basenempfindlichen Isocyanate.

Die US-Patentschriften 3 372 180 sowie 3 392 184 beschreiben Verfahren zur Darstellung von 2,4-disubstituierten Allophansäureestern sowie ihre thermische Zersetzung zu Isocyanaten und Hydroxyverbindungen. Diese Reaktion liefert jedoch nur dann befriedigende Ausbeuten, wenn als Pyrolysekomponenten N,N'-Dialkylallophansäurearylester verwendet werden, deren Esterteil einem hochsiedenden Phenol entspricht. Bereits beim Übergang von N,N'-Dimethyl-2,6-di-t-butyl-4-methylphenylallophanat (Kp. 2,6-Di-t-butyl-4-methylphenol: 265°C) zum N,N'-Dimethyl-3-t-butylphenylallophanat (Kp. 3-t-Butylphenol: 240°C) fällt die Ausbeute an Methylisocyanat um ca. 20% ab. Beim weiteren Übergang zum N,N'-Dimethylallophansäurephenylester (Kp. Phenol: 182°C) fällt die Isocyanatausbeute noch weiter ab (Vergleichsbeispiel 5).

Es wurde nun gefunden, dass man aliphatische Isocyanate der Formel

$R^1$-NCO I,

worin $R^1$ einen aliphatischen Rest bezeichnet, durch thermische Zersetzung von N,N'-disubstituierten Allophansäureestern der Formel

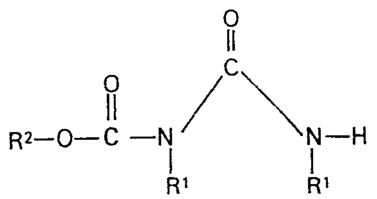
II,

worin die einzelnen Reste $R^1$ gleich oder verschieden sein können und die vorgenannte Bedeutung besitzen, $R^2$ einen aromatischen Rest, dessen unsubstituiertes oder substituiertes Phenol bei einer Temperatur von unterhalb 250°C destillierbar ist, bedeutet, erhält, wenn man die Ausgangsstoffe II in Gegenwart von Diestern der Kohlensäure der Formel

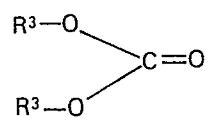
III,

worin die einzelnen Reste $R^3$ gleich oder verschieden sein können und einen aliphatischen, cycloaliphatischen, araliphatischen, aromatischen Rest bedeuten oder zusammen für einen unsubstituierten oder substituierten Alkylenrest

oder Phenylenrest stehen, bei einer Temperatur von 150 bis 400°C thermisch zersetzt.

Die Umsetzung kann beispielsweise im Falle der Verwendung von N,N'-Dimethylallophansäurephenylester und Diphenylcarbonat als Reaktionsmedium durch die folgenden Formeln wiedergegeben werden:

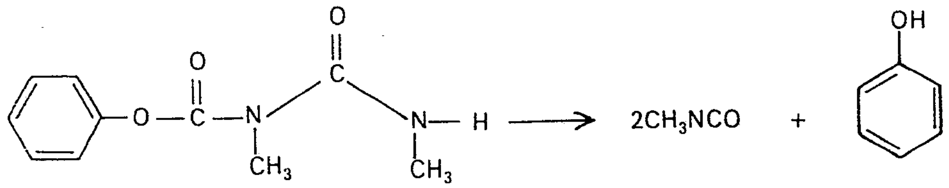

Im Vergleich zu dem bekannten Verfahren liefert das Verfahren nach der Erfindung überraschend auf einfacherem und wirtschaftlicherem Wege eine grosse Zahl von aliphatischen Isocyanaten in wesentlich besserer Ausbeute, Raum-Zeit-Ausbeute und Reinheit, gerade auch im grosstechnischen Massstab und im kontinuierlichen Betrieb. Alle diese vorteilhaften Ergebnisse sind überraschend, da man angesichts der Anwesenheit von dem sehr reaktiven Ausgangsstoff III, den verschiedenen Umsetzungsmöglichkeiten des Ausgangsstoffes II und bei der hohen Temperatur ein Gemisch zahlreicher Komponenten und eine geringere Ausbeute und Reinheit des Endstoffs I hätte erwarten müssen. Hinsichtlich US-PS 3 275 669, 3 372 180 sowie 3 392 184 überraschen die hohen Ausbeuten unter Verwendung der Ausgangsstoffe III.

Bevorzugte Ausgangsstoffe II und III und dementsprechend bevorzugte Endstoffe I sind solche, in deren Formeln die einzelnen Reste $R^1$ gleich oder verschieden sein können und jeweils einen Alkylrest mit 1 bis 7 oder einen Alkenylrest mit 2 bis 7 Kohlenstoffatomen bedeuten, und/oder $R^2$ für einen unsubstituierten oder durch mehrere oder zweckmässig 1 oder 2 Fluoratome, Chloratome, o-Nitrogruppen, Alkylgruppen, Alkoxygruppen mit jeweils 1 bis 4 Kohlenstoffatomen, substituierten Phenylrest, dessen Phenol bei Temperaturen von unterhalb 250°C destillierbar ist, steht, $R^3$ einen Alkylrest mit 1 bis 7 Kohlenstoffatomen, einen Alkenylrest mit 2 bis 7 Kohlenstoffatomen, einen Arylalkylrestoder Alkylarylrest mit jeweils 7 bis 12 Kohlenstoffatomen oder einen Phenylrest bezeichnet oder die 2 Reste $R^3$ zusammen Glieder eines unsubstituierten oder substituierten Alkylenrestes mit 2 bis 6 Kohlenstoffatomen oder eines unsubstituierten oder substituierten Phenylrestes mit 6 bis 12 Kohlenstoffatomen stehen kann. Die vorgenannten Reste können durch unter den Reaktionsbedingungen inerte Gruppen, z.B. Nitrogruppen, Alkyl- oder Alkoxygruppen mit jeweils 1 bis 4 Kohlenstoffatomen, substituiert sein.

So kommen folgende Ausgangsstoffe II in Betracht: N,N'-Dimethylallophansäurephenylester; die homologen N,N'-Diäthyl-, N,N'-Dipropyl-, N,N'-Diisopropyl-, N,N'-Dibutyl-, N,N'-Diisobutyl-, N,N'-Di-tert.-butyl-, N,N'-Di-sek.-butylamin-, N-Methyl-, N'-Äthylverbindungen des vorgenannten Allophansäureesters; an den beiden Stickstoffatomen durch vorgenannte Substituenten substituierte, und durch 1 oder 2 Methyl-, Äthyl-, Propyl-, Isopropyl-, Butyl-, Isobutyl-, tert.-Butyl-, sek.-Butyl-, Allyl-, Nitro-, Methoxy-, Alkoxy-, Propoxygruppen, Fluoratome, Chloratome am Phenylkern des Phenols in 2-, 3-, 4-Stellung einfach oder in 2',6'-, 2',3'-, 2',4'- 2',5'-, 3',5'-, 4',5'-Stellung gleich oder unterschiedlich zweifach substituierte Phenole.

Als Ausgangsstoff III sind beispielsweise geeignet: Diallyl-, Dibutyl-, Di-N-butyl-, Diisobutyl-, Di-tert.-Butyl-, Di-sek.-butyl-, Dicyclohexyl-, Dibenzyl-, Diphenyl-ester der Kohlensäure; cyclische Carbonate wie Äthylen-, 1,2-Propylen-, 1,3-Propylen-, 1,4-Butylen-, 1,2-Butylen-, Isobutylen-, Phenylen-(1,2)-, Phenylen-(1,3). Bevorzugt sind Ausgangsstoffe III mit einem Siedepunkt oberhalb 150°C, insbesondere Diphenyl-, Di-α-naphthyl, Di-p-nitrophenyl-, Di-n-decyl-, Di-o-Kresyl, Di-m-Kresyl, Di-p-Kresylcarbonat.

Bezogen auf den Ausgangsstoff II, kann der Ausgangsstoff III in stöchiometrischer Menge oder im Überschuss, vorzugsweise in einem Verhältnis von 0,01 bis 10, insbesondere 0,1 bis 5 Mol Ausgangsstoff II je Mol Ausgangsstoff III, umgesetzt werden. Die Umsetzung wird im allgemeinen bei einer Temperatur von 150 bis 400°C, vorzugsweise von 180 bis 360°C, mit Unterdruck, Überdruck oder drucklos, vorzugsweise drucklos oder mit Unterdruck, vorzugsweise von 200 bis 1000 mbar, diskontinuierlich oder kontinuierlich, durchgeführt.

Die Reaktion kann wie folgt durchgeführt werden: Ein Gemisch von Ausgangsstoff II und Ausgangsstoff III wird während 0,1 bis 2 Stunden bei der Reaktionstemperatur gehalten. Innerhalb dieser Zeit wird aus dem Reaktionsgemisch der Endstoff in üblicher Weise, z.B. durch Destillation, abgetrennt. Vorteilhaft sind die neutralen Reaktionsbedingungen sowie die Abwesenheit von Katalysatoren und damit kein Salzanfall und Umweltfreundlichkeit, da neben den erfindungsgemässen Isocyanaten nur Phenole gebildet werden, welche quantitativ zur Darstellung der Allophanate wieder verwendet werden können. Gegenüber dem in der Technik gebräuchlichen Verfahren zur Darstellung von Isocyanaten wird durch das erfindungsgemässe Verfahren eine deutliche Verminderung des Phosgenverbrauchs erreicht.

Die nach dem Verfahren der Erfindung herstellbaren Isocyanate sind wertvolle Ausgangsstoffe für die Herstellung von Pflanzenschutzmitteln, Schädlingsbekämpfungsmitteln, Farbstoffen, Kunstharzen und Kunststoffen, Textilhydrophobierungsmitteln, Waschmitteln, Bleichmitteln und Klebstoffen. Insbesondere sind ihre Umsetzungen zu Urethanen, z.B. für die Verwendung als Schaumstoffe oder hochmolekulare Überzüge mit hoher Flexibilität, oder Harnstoffen von Bedeutung. Bezüglich der Verwendung wird auf vorgenannte Veröffentlichungen und Ullmanns Encyklopädie der technischen Chemie, Band 9, Seiten 11, 12, 404, und Band 17, Seite 204, verwiesen.

Die in den folgenden Beispielen aufgeführten Teile bedeuten Gewichtsteile. Sie verhalten sich zu den Volumenteilen wie Kilogramm zu Liter.

Beispiel 1

700 Gewichtsteile N,N'-Dimethylallophansäurephenylester und 500 Gewichtsteile Di-p-nitrophenylcarbonat werden auf 240°C erhitzt. Die Reaktionszeit beträgt 90 Minuten. Das bei der Umsetzung entstehende Methylisocyanat wird am Kopf einer aufgesetzten Kolonne entnommen. Das Phenol wird am unteren Teil der Kolonne abgenommen. Man erhält 376 Teile Methylisocyanat (98% der Theorie) vom Kp 39–41°C.

Beispiel 2

700 Gewichtsteile N,N'-Dimethylallophansäurephenylester und 500 Gewichtsteile Di-α-naphthylcarbonat werden auf 225°C erhitzt. Die Reaktionszeit beträgt 90 Minuten. Das bei der Umsetzung entstehende Methylisocyanat wird am Kopf einer aufgesetzten Kolonne entnommen, das Phenol am unteren Teil der Kolonne abgenommen. Man erhält 361 Teile Methylisocyanat (97,1% der Theorie) vom Kp 39–41°C.

Beispiel 3

104 Gewichtsteile N,N'-Dimethylallophansäurephenylester und 100 Gewichtsteile D-n-decylcarbonat werden auf 240°C erhitzt. Die Reaktionszeit beträgt 90 Minuten. Das bei der Umsetzung entstehende Methylisocyanat wird am Kopf einer aufgesetzten Kolonne entnommen, das Phenol am unteren Teil der Kolonne abgenommen. Man erhält 55 Teile Methylisocyanat (96,5% der Theorie) vom Kp 39–41°C.

Beispiel 4

70 Gewichtsteile N,N'-Dimethylallophansäurephenylester und 80 Gewichtsteile Diphenylcarbonat werden im Ölbad auf 240°C erhitzt. Die Reaktionszeit beträgt 90 Minuten. Das bei der Umsetzung entstehende Methylisocyanat wird am Kopf einer aufgesetzten Kolonne entnommen, das Phenol am unteren Teil der Kolonne abgenommen. Man erhält 35 Teile Methylisocyanat (91% der Theorie) vom Kp 39–41°C.

Beispiel 5

(Vergleichsbeispiel)

70 Gewichtsteile N,N'-Dimethylallophansäurephenylester werden im Ölbad auf 240°C erhitzt. Die Reaktionszeit beträgt 90 Minuten. Das bei der Umsetzung entstehende Methylisocyanat wird am Kopf einer aufgesetzten Kolonne entnommen, das Phenol am unteren Teil der Kolonne abgenommen. Man erhält 17,9 Teile Methylisocyanat (45,6% der Theorie) vom Kp 39–41°C.

Beispiel 6

47 Gewichtsteile N,N'-Diäthylallophansäurephenylester und 80 Gewichtsteile Diphenylcarbonat werden im Ölbad auf 240°C erhitzt. Die Reaktionszeit beträgt 90 Minuten. Das bei der Reaktion entstehende Äthylisocyanat (Kp 60°C) wird am Kopf einer aufgesetzten Kolonne entnommen, das Phenol am unteren Teil der Kolonne abgenommen. Man erhält 26,2 Teile Ethylisocyanat (94,6% der Theorie) vom Kp 60°C.

Beispiel 7

(Vergleichsbeispiel)

50 Gewichtsteile N,N'-Diäthylallophanat werden im Ölbad auf 240°C erhitzt. Die Reaktionszeit beträgt 90 Minuten. Das bei der Umsetzung entstehende Äthylisocyanat wird am Kopf einer aufgesetzten Kolonne entnommen, das Phenol am unteren Ende der Kolonne abgenommen. Man erhält 15,3 Teile Ethylisocyanat (50,3% der Theorie) vom Kp 60°C.

Beispiel 8

51 Gewichtsteile N,N'-Di-n-propylallophansäurephenylester und 70 Gewichtsteile Diphenylcarbonat werden auf 240°C erhitzt. Die Reaktionszeit beträgt 90 Minuten. Das bei der Umsetzung entstehende n-Propylisocyanat wird am Kopf einer aufgesetzten Kolonne entnommen, das Phenol am unteren Ende der Kolonne abgenommen. Man erhält 31,7 Teile n-Propylisocyanat (98,5% der Theorie) vom Kp 88°C.

Beispiel 9

50 Gewichtsteile N,N'-Dimethylallophansäure-3-t-butylphenylester und 83 Gewichtsteile Diphenylcarbonat werden im Ölbad bei 240°C während 90 Minuten erhitzt. Das bei der Umsetzung entstehende Methylisocyanat wird am Kopf einer aufgesetzten Kolonne entnommen, das Phenol am unteren Ende der Kolonne abgenommen. Man erhält 20,6 Teile Methylisocyanat (95% der Theorie) vom Kp 39–41°C.

**Patentanspruch**

Verfahren zur Herstellung von aliphatischen Isocyanaten der Formel

$R^1$-NCO I,

worin $R^1$ einen aliphatischen Rest bezeichnet, durch thermische Zersetzung von N,N'-disubstituierten Allophansäureestern der Formel

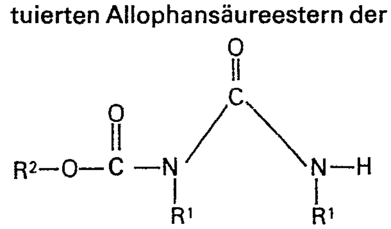

II,

worin die einzelnen Reste $R^1$ gleich oder verschieden sein können und die vorgenannte Bedeutung besitzen, $R^2$ einen aromatischen Rest, dessen unsubstituiertes oder substituiertes Phenol bei einer Temperatur von unterhalb 250°C destillierbar ist, bedeutet, dadurch gekennzeichnet, dass man die Ausgangsstoffe II in Gegenwart von Diestern der Kohlensäure der Formel

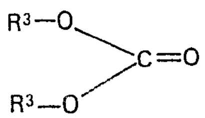
III,

worin die einzelnen Reste $R^3$ gleich oder verschieden sein können und einen aliphatischen, cycloaliphatischen, araliphatischen, aromatischen Rest bedeuten oder zusammen für einen unsubstituierten oder substituierten Alkylenrest oder Phenylenrest stehen, bei einer Temperatur von 150 bis 400°C thermisch zersetzt.

**Claim**

A process for the preparation of an aliphatic isocyanate of the formula

$R^1$-NCO I,

where $R^1$ is an aliphatic radical, by thermal cleavage of an N,N'-disubstituted allophanic acid ester of the formula

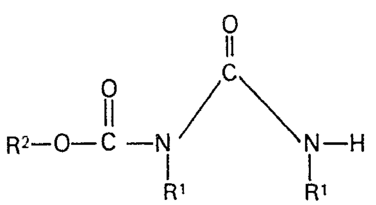
II,

where the $R^1$'s may be identical or different and have the above meanings and $R^2$ is an aromatic radical derived from an unsubstituted or substituted phenol which is distillable at below 250°C, wherein the starting material II is thermally cleaved at from 150 to 400°C in the presence of a diester of carbonic acid, of the formula

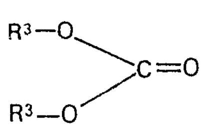
III,

where the $R^3$'s may be identical or different and each is an aliphatic, cycloaliphatic, araliphatic or aromatic radical, or the $R^3$'s together are an unsubstituted or substituted alkylene or phenylene radical.

**Revendication**

Procédé pour la préparation d'isocyanates aliphatiques de formule

$R^1$-NCO (I)

dans laquelle $R^1$ représente un radical aliphatique, par décomposition thermique d'esters d'acide allophanique N,N'-disubstitués de formule

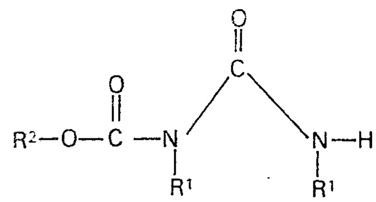
II,

dans laquelle les radicaux $R^1$ peuvent être semblables ou différents et ont la signification donnée ci-dessus, $R^2$ représente un radical aromatique, dont le phénol non substitué ou substitué est distillable à une température de moins de 250°C, caractérisé en ce qu'on décompose thermiquement les composés de départ II, à une température de 150 à 400°C, en présence de diesters de l'acide carbonique de formule

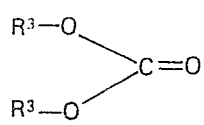
III,

dans laquelle les radicaux $R^3$ peuvent être semblables ou différents et représentent chacun un radical aliphatique, cycloaliphatique, araliphatique, aromatique ou forment ensemble un radical ou phénylène non substitué ou substitué.